Europäisches Patentamt

European Patent Office

Office européen des brevets

(11) Publication number: **0 112 915**
**B1**

(19)

# (12) EUROPEAN PATENT SPECIFICATION

(45) Date of publication of patent specification: **15.10.86**

(51) Int. Cl.⁴: **A 23 K 1/00,** A 61 K 9/32, A 61 K 9/42

(21) Application number: **83902492.4**

(22) Date of filing: **06.07.83**

(86) International application number:
**PCT/US83/01011**

(87) International publication number:
**WO 84/00282 02.02.84 Gazette 84/03**

(54) **RUMEN-STABLE PELLETS.**

(30) Priority: **12.07.82 US 397314**
**26.05.83 US 498446**

(43) Date of publication of application:
**11.07.84 Bulletin 84/28**

(45) Publication of the grant of the patent:
**15.10.86 Bulletin 86/42**

(84) Designated Contracting States:
**CH DE FR GB LI NL**

(56) References cited:
**GB-A- 872 149**
**US-A-3 981 984**
**US-A-4 181 708**

**Chemical Abstracts, volume 89, no. 18, October
1978, Columbus, Ohio (US) see page 31,
column 2, abstract 147656q**

(73) Proprietor: **EASTMAN KODAK COMPANY**
**343 State Street**
**Rochester New York 14650 (US)**

(72) Inventor: **WU, Stephen, Hong-Wei**
**1104 Meadow Lane**
**Kingsport, TN 37663 (US)**
Inventor: **SANDHU, Mohammad, Akram**
**2600 Wildwood Drive**
**Kingsport, TN 37660 (US)**

(74) Representative: **Parent, Yves et al
Kodak-Pathé Département Brevets et Licences
Centre de Recherches et de Technologie Zone
Industrielle
F-71102 Chalon-sur-Saone Cédex (FR)**

Courier Press, Leamington Spa, England.

## Description

This invention relates in general to methods for making pellets adapted to be orally administered to ruminants. More particularly, this invention relates to methods for making pellets having a core material such as a nutrient or medicament, and a coating over the core material which protects the core in the environment of the rumen. The coating loses continuity under the more acidic conditions of the abomasum to render the core material available for utilization by the animal.

In ruminants, e.g., beef and dairy cattle, sheep, etc., ingested feed first passes into the rumen, where it is pre-digested by fermentation. During this period of fermentation the ingested feed is subjected to rumination, which is part of a digestive process involving regurgitation. After a period of fermentation, adsorption of digested nutrients starts and continues in the subsequent sections of the digestive tract. This digestive process is described in detail by D. C. Church, "Digestive Physiology and Nutrition of Ruminants," Vo. 1, O.S.U. Book Stores, Inc., of Corvallis, Oregon.

In preparing nutrients and medicaments intended for administration to ruminants, it is important to protect the active ingredients against the environmental conditions of the rumen, i.e., microbial degradation and the effects of a pH in the range of 5.5 to 7.0. If so protected, the active substance will be saved until it reaches the particular location where adsorption takes place. It is well known that the rate of meat, wool and/or milk production can be increased if sources of growth limiting essential amino acids, and/or medicaments, are protected from alteration by microorganisms residing in the rumen and become available for direct adsorption by the animal later in the gastrointestinal tract.

Because proteins are subject to breakdown in the rumen, it has been suggested that protein-containing nutrients fed to ruminants be treated so as to permit passage without microbial breakdown through the rumen to the abomasum. Suggested procedures have included coating the protein material, for example, with fats and vegetable oils; heat treating of the protein material; reacting the protein material with various compounds such as formaldehyde, acetylenic esters, polymerized unsaturated carboxylic acid or anhydrides and phosphonitrilic halides, etc.

The pellets described in U.S. Patent 4,181,708 have a core comprising a nutrient and/or medicament and the desired coating on the core. The coating protects the core in the environment of the rumen. The continuity of the coating is destroyed in the more acidic environment of the abomasum thereby releasing the core material. This patent discloses coatings comprising a polymer, hydrophobic substance and flake material, but does not disclose treatment of the flake material.

These pellets are highly desirable ruminant feeds. However, further improvements have been sought in the rumen protection provided by the pellet coating. An improved coating could provide either the same rumen protection using less coating material or greater protection using the same amount of coating material as was used in the pellets described in U.S. Patent 4,181,708. Thus, the problem to be solved was to provide a coating material which provides greater rumen protection. The present method solves this problem.

More particularly, the present invention provides a method for making coated pellets comprising a core material and a coating, the coating composition comprising a film-forming polymeric material, a hydrophobic material and flake particles, the method comprising coating the core material with the coating composition, characterized in that prior to coating, the flake particles are brought into rubbing contact with the hydrophobic material.

According to the present invention, the flake material is treated by bringing the particles thereof into rubbing contact with the hydrophobic material, preferably in a liquid medium, for a sufficient time to cause the flake material to become more hydrophobic. Generally, from 2 to 28 hours under at least light shearing stress and/or direct pressure (0.1 to 20 $Kg/cm^2$) is sufficient. In this manner, the surface energy of the flake material is changed such that it becomes more hydrophobic. It has been found that rumen protection can be substantially improved by this special treatment of the flake material.

To cause the rubbing contact between the flake material and the hydrophobic material, it is preferred that they be ball-milled. However, other conventional processes and equipment can be used. For example, other milling processes, grinding, vigorous mixing, and the like may be used. Vehicles, such as mineral spirits, volatile hydrocarbons and ketones, may be used to disperse the hydrophobic material and flake material if desired.

A preferred ball mill used in the practice of this invention comprises a cylindrical container mounted horizontally and partially filled with ceramic balls. The surface modification of the flake material with hydrophobic substances in acetone is accomplished by rotating the ball mill and its contents about the horizontal axis of the mill at a rate sufficient to lift the balls to one side and then cause them to roll, slide, and tumble (cascade) to the lower side.

A laboratory procedure practiced in this invention is described as follows:

1. Weight approximately 570 g of ceramic balls and add these balls into a ball mill. The total volume of the balls is approximately 15—25% of the volume of the cylindrical container.

2. Add 137 g of a mixture of flake material and hydrophobic substance, and 320 mL acetone to the ball mill, and seal the container to minimize evaporation of acetone during the operationm.

3. Rotate the ball mill on a roller at a speed of ~90 revolutions per minute for 16 hrs.

4. Decant the contents and wash the container and balls with acetone. The disperson is ready to be used for the preparation of a coating composition, or the disperson is dried by evaporating acetone at 50°C. The dry mixture of flake material/ hydrophobic substance is then redispersed into a polymer solution to make a coating composition.

In the practice of this invention, the polymeric material may conveniently be dissolved in a suitable organic solvent which would be physiologically acceptable in the event there are residues upon evaporation of the solvent. The hydrophobic substance and flake material are blended in the solution, wherein the polymeric substance is a continuous matrix and the additives are dispersed therein. The coating solution may be applied by various well known means such as, for example, brushing, dipping, spraying, fluidized bed, etc.

To illustrate the present invention, a sample of poly(2-methyl-5-vinylpyridine/styrene, 80/20) can be dissolved in acetone and a portion of the flake material/hydrophobic substance/acetone dispersion or a dry mixture of flake material/ hydrophobic substance is added to it with stirring. The dispersion is vigorously mixed for 40 minutes. A small amount of water (less than 8%) may be added to the disperson to minimize the electrostatic charges during the coating process.

An air-suspension coater may be used for spray coating nutrient particles containing methionine, glucose, lysine, or other active ingredients. The typical coating temperatures are 50°C and 32°C for inlet and outlet air, respectively. In a typical coating operation, heated air at 0.7 m³/min. at 1 kg/cm² is admitted to the chamber of the coater to cause the nutrient particles to circulate. The coating dispersion is pumped by a precision pump at a rate of 20 mL/min and atomized through the nozzle by compressed air at 0.15 m³/min. and 3 kg/cm² continuously. The product is in the form of pellets encapsulated with flaked polymeric coating.

The coating weight is determined by dispersing and rinsing one gram of coated pellets with 50 mL acetone for several times until coatings were dissolved. The weight loss from coated pellets is measured to calculate the percent coating weight.

The pellets made according to this invention are adapted for oral administration to ruminants. The pellets are preferably of a suitable size, such as between No. 10 and No. 18 of U.S. Standard Sieve Size. Also, the pellets must be of suitable density, preferably having a specific gravity of between 1 and 1.4, and preferably have acceptable odor, taste, feel, etc. The pellets include a core and a continuous, film or coating completely encapsulating the core. The pellets are preferaly cylindrical, having a diameter of 1.0—2.0 mm and a length-to-diameter ratio of 1—2.0:1, or spherical, having a diameter of 1.0—3.0 mm.

The core is of a material beneficial to the ruminant upon passing the rumen and reaching the abomasum and/or intestine. Normally, the core is a solid material which has been formed into particles, such as by pelletizing. The core should have sufficient body or consistency to remain intact during handling, particularly during the coating operation. Suitable core materials include various medicaments and nutrients such as, for example, antibiotics, relaxants, drugs, antiparasites, amino acids, proteins, sugars, carbohydrates, etc. The core may also contain inert filler material such as clay. Other suitable core materials are described in U.S. Patent 4,181,708, mentioned above.

The coating composition is capable of forming a continuous film around the core by the evaporation of solvent from the coating composition. The coating composition material is preferably resistant to pH conditions of greater than 5 for from 6 to 30 hours. The coating composition preferably releases the core material after exposure to abomasum environmental conditions having a pH of 2 to 3.3. Release occurs within the residence time in the abomasum or later in the intestinal tract but at least within a time period of 6 hours after contacting pH 3.5 or less. The exposure of the core may occur by the coating becoming permeable to the contents of the rumen, such as by dissolving, disintegrating, or extensive swelling. The coating composition is physiologically acceptable, i.e., the coating composition should not interfere with the ruminants' healthy or normal body functioning.

The coating composition is able to withstand abrasion in handling and storage conditions of relatively high heat and/or humidity without a significant amount of blocking or sticking. It preferably has a sticking temperature of greater than 50°C. Sticking temperature is defined as the temperature at which an applied force of 0.25 kg/ cm² for 24 hours causes the coating of pellets to adhere to the coating of adjacent pellets strongly enough to cause rupture of the coating when the pellets are forceably separated. Also, the coating composition is preferably soluble or dispersable in organic solvents having boiling points of between 40°C and 140°C to permit conventional coating processes such as spray coating to be used. Particularly suitable solvents include methylene chloride, chloroform, ethanol, methanol, ethyl acetate, acetone, toluene, isopropanol or mixtures of these.

The coating composition includes a mixture or blend of at least one polymeric substance, at least one hydrophobic substance, and at least one flake material. Generally, the more acidic and more soluble core materials require greater ratios of hydrophobic substance and flake material to polymeric substance, while more basic and less soluble core materials require lesser ratios of hydrophobic substance and flake material to polymeric substance within the range. The hydrophobic substance and flake material are normally dispersed in the polymeric matrix. The coating normally contains 16—87% by weight polymeric material, 0.5—32% by weight hydrophobic substance and 7—80% by weight flake material.

Suitable polymers for the coating composition are described in U.S. Patent 4,181,708, mentioned above. Especially preferred polymers are poly-(vinylpyridine), polymeric derivatives of vinylpyridine, and the copolymers of the various isomers and derivatives of vinylpyridine copolymerized with one or more addition type monomers. Especially preferred are copolymers of 2-methyl-5-vinylpyridine and styrene, and in particular, the copolymer of 75—85% by weight 2-methyl-5-vinylpyridine and 15—25% by weight styrene, as well as the copolymer of 55—65% by weight 2-methyl-5-vinylpyridine and 35—45% by weight acrylonitrile. Also especially preferred is a copolymer of 75—85 wt. % 2-vinylpyridine and 25—15 wt. % styrene. These copolymers are commercially available or may be produced by conventional techniques well known in the art.

Hydrophobic substances which are physiologically acceptable and have the correct degree of compatibility with the polymer are commercially available. It is important that the polymer and hydrophobic substance have a degree of compatibility to permit the film to remain intact in the rumen environment, but to permit permeation of the abomasal fluid to the core while the pellet is in the abomasum.

A class of hydrophobic substances of value are fatty acids containing from 10 to 32 carbon atoms such as lauric, oleic, stearic, palmitic and linoleic. These substances are well known to be water insoluble due to the long hydrocarbon radical but to react to water due to the polar nature of the carboxyl group. In the selected basic amino group-containing polymers, the carboxyl group of the fatty acid is able to react with the basic nitrogen group to form a weak salt-type linkage. This attachment to the polymer serves to cause the fatty acid to be fixed in the polymer matrix. The hydrophobic hydrocarbon chain of the fatty acid tends to render the matrix water resistant and thereby decreases swelling of the otherwise water susceptible polar film. Both the interior of the matrix film and the surface is now water resistant in aqueous environments at pH above 5.0. However, at pH values below pH 4.5 and especially below pH 3.5 the affinity of the basic nitrogen group for water and the hydrogen ion overcomes the increased water resistance. The film reacts with the acid environment and loses barrier properties sufficient to allow the core material to escape to the environment.

Polyfunctional carboxylic acids may be derived from natural products or obtained by organic synthesis but the ratio of carboxyl group to hydrophobic organic radical should be at least 1 to 10 based on the molecular weight of the organic radicals. Also included in this class of synthesized organic hydrophobic acids are mono and polyfunctional acids containing silicone or fluorinated carbon groups located at least 4 atoms distant along the molecular chain from the position of the carboxyl group or groups. Also, included in the class of hydrophobic substances are the nontoxic multivalent metallic salts of the above acids such as the stearates, oleates, fatty acid dimerates, and palmitates of aluminum and iron and the calcium, magnesium and zinc salts of the higher molecular weight crystalline analogs of the above acids. When the cation is trivalent as for aluminum and ferric iron, the molar ratio of organic acid to metal ion is 2 to 1 or 3 to 1 and the acid can be any monofunctional organic acid having one carboxyl group and at least 10 carbon atoms in the organic radical attached to the carboxyl group. When the metal ion is divalent such as ferrous iron, calcium, magnesium or zinc the organic acid may be monocarboxylic or polycarboxylic and the ratio of metal ion to noncarboxylic carbon atoms is at least 1 to 26. Natural and synthetic waxes and resins added at levels depending on the degree of hydrophobicity and compatibility in the matrix film are of value in the practice of the invention. Waxes and resins are useful that have a critical surface tension of less than 31 dynes/cm as determined by the Zisman method described in "Contact Angle Wettability and Adhesion," Advances in Chemistry Series No. 43; Edited by Robert F. Gould; published by the American Chemical Society; 1963; Chapter 1; and have a solubility in the matrix film of less than 5%. These waxes and resins are dispersed in the film in at least amounts equal to 2 times the solubility and up to 30% of the total weight of the matrix polymer. Typical waxes and resins include beeswax, petroleum wax, dammar, hard manila, phenolic resins and maleated low molecular weight polyhydrocarbons. Also included in the hydrophobic substances are polymers having weight average molecular weights of from 2000 to 1,000,000, a critical surface tension of less than 31 dynes/cm measured by methods in the reference to Zisman described above. Useful polymers have a solubility or compatibility in the matrix film of less than 5% on a weight basis and are present in the film at levels at least equal to two times the solubility and up to 30 weight percent of the matrix film. Of particular value are the polymers and copolymers containing silicone groups in the main polymer chain or in a side chain and polymers and copolymers containing fluorinated carbon groups in a side chain. Regardless of the exact nature of the hydrophobic substance it must be soluble or colloidally dispersible in the coating solvent when one is used. The hydrophobic substance makes up from 1 to 50% of the combined weight of polymeric material and hydrophobic substance.

Suitable hydrophobic substances also include fatty acids having from 12 to 32 carbon atoms, such as oleic acid and stearic acid, dimer acids, trimer acids, aluminum salts of fatty acids, waxes, resins, and certain polymers such as polymers containing very hydrophobic chemical groups such as silicone moieties and certain multivalent cation soaps. The hydrophobic substance may be amorphous or crystalline and preferably essentially dispersible in the coating solvent when a solvent is used in which case it should not contribute significantly to the solution viscosity.

Aluminum salts of such acids, for example, aluminum oleates, aluminum stearates, aluminum dimerates, are also useful. Also, the hydrophobic material may be one or more polycarboxylic acids having a ratio of from 10 to 22 carbon atoms per carboxyl group are useful. Blends of these acids and/or salts are also useful.

Suitable inert flake materials include metal flake, mineral flake, crosslinked organic polymer, etc. Especially suitable are aluminum flake, talc, graphite, and ground mica. A preferred flake material is a blend of aluminum and talc.

Aluminum flake is produced by ball-milling the aluminum in a liquid medium in the presence of a lubricant such as stearic acid. It is available commercially from Alcon Metal Powders, Division of Alcon Aluminum Corporation. Sizes of the flake are generally less than 100 microns, preferably about 1—60 microns. Talc particle sizes are generally within the range of 0.5—40 microns.

In preferred embodiments the coating composition comprises

(a) from 16 to 87% based on the total composition weight of a film-forming polymeric material,

(b) from 0.5 to 32%, based on the total composition weight of a hydrophobic material dispersed in said polymeric material, and

(c) from 7 to 80%, based on the total composition weight, of a flake material dispersed in said polymeric material.

The polymeric material in combination with said hydrophobic material and flake material is physiologically acceptable and resistant to a pH of greater than 5 but capable of releasing the core of the pellets at a pH of less than 3.5, the coating preferably having a sticking temperature of at least 50°C, as previously described.

The performance of the coated pellets is evaluated by simulated *in vitro* rumen protection and abomasal release tests.

The protection test mentioned in the examples is done by agitating one gram of coated pellets in sodium acetate buffer at pH 5.4 for 24 hrs. in a 37°C water bath. The release test is done by extracting one gram of pellets in sodium citrate buffer at pH 2.9 for 1 hr. in a 37°C water bath. The supernatants taken from different media are centrifuged to remove undissolved pellets and other insoluble materials. Methionine and chloride ion concentration in the supernatants from coated methionine and lysine.HCl pellets, respectively, are determined by a X-ray fluorescence method. Glucose concentrations in the supernatants are determined by either a gravimetric method or a colorimetric method. The percent protection is calculated from the difference of the initial amount of active ingredient in the coated pellets and the amount of active ingredients released to the media.

The examples which follow are submitted for a better understanding of the invention. While the examples are based on *in vitro* tests, the *in vitro* experiments shown in the examples simulate conditions existing in ruminants thereby permitting the study of coated pellets without the use of live animals. It has been determined by actual *in vivo* tests that the testing of pellets in the aqueous media used in the examples, simulating the environmental conditions of the rumen and abomasum with respect to temperature, pH, etc., provide reliable data concerning the protection offered by the coatings in the rumen, and releasability of the coatings in the abomasum. Nutrients such as amino acids and proteins which may be used in the core material are known to be beneficial to ruminants when positioned in the intestinal tract downstream from the rumen.

Generally, pellets are prepared from the nutrients indicated to a size of between 10 and 18 sieve size. The nutrients are mixed with conventional additives such as microcrystalline cellulose, binders, inert consistency adjusting substances such as water, etc. The pellets are formed by a conventional pelletizer, dried, sieved, and coated using a coater as described herein. Upon formation of an imperforate coating on the pellets, they are tested for resistance to pH conditions resembling those of the rumen and abomasum by agitating in buffer solutions of pH 2.9 for 0.5 hours and 5.4 for 24 hours. Recovery and protection figures cited for active core ingredients herein contain in them all materials of the original coated pellet that are not completely dissolved in the pH 2.9 buffer, including any undissolved active ingredient in the original core.

### Example 1

Methionine cores (−12/+16 sieve size, U.S. standard. −12/+16 Means that the cores will pass through a No. 12 sieve, 1.68 mm openings, but not through a No. 16 sieve, 1.19 mm openings) were coated with a coating composition composed of poly(2-methyl-5-vinylpyridine/styrene, 80/20) [hereinafter (2M5VP/ST)]/talc/aluminum/ calcium stearate (31.5/38.1/25.4/5.0, by wt.). Talc, aluminum flake, and calcium stearate were ball-milled at ~40% weight per volume (w/v) in acetone and then mixed with the polymer solution to make a 5% solids coating composition. Pellets were coated using an air suspension coater as described previously. Protection values for pellets coated with the surface modified flake formulations showed improvement over the control pellets without ball-milling treatment. It requires only 8.5% coating wt. of ball-milled flake material to provide 90% methionine protection, whereas, it takes 13% coating wt. for the control pellets to reach the same value.

### Example 2

Methionine cores (−12/+16 sieve size) were coated with a coating composition composed of (2M5VP/ST)/talc/aluminum/stearic acid (31.5/38.1/ 25.4/5.0, by wt.). Talc, aluminum flake, and stearic acid were ball-milled at 40% w/v in acetone and then mixed with the polymer solution to make a 5% solid coating composition. Pellets were coated as described previously. The protection and efficiency of the ball-mill-treated coating showed improvement over the untreated control at all coating levels.

## Example 3

Methionine cores (−12/+16 sieve size) were coated with a coating composition composed of (2M5VP/ST)/talc/stearic acid (31.5/65/3.5). Talc and stearic acid were ball-milled in acetone, dried, and redispersed in the polymer solution in preparing the coating composition. Results showed that the methionine protection of coated pellets with treated coatings was higher than the protection values of coated pellets without ball-milling treatments. Release of the methionione was not affected by the ball-milling treatment.

## Example 4

Glucose cores (−10/+12 sieve size) were coated with a coating composition composed of (2M5VP/ST)/talc/stearic acid (31.5/63.6/4.9). Talc and stearic acid were ball-milled in acetone, dried and redispersed in the polymer solution in preparing the coating composition. Glucose protection was 95% at pH 5.4 for pellets coated with 14% coating, and the release was completed at pH 2.9 in 1 hr. The protection values were comparable with the values for pellets coated with a coating of (2M5VP/ST)/talc/aluminum/stearic acid, 31.5/39/26/3.5, described previously.

## Examples 5—7

Glucose pellets (−12/+14 sieve size) were coated with a series of coating compositions composed of 31.5% (2M5VP/ST), 38.1% talc, 25.4% aluminum flake, and 5% stearic acid. Talc, aluminum, and stearic acid were ball-milled in acetone, dried and redispersed in the polymer solution in preparing the coating composition. In another preparation, talc and aluminum, without added stearic acid, were ball-milled and then dried and redispersed in a polymer solution.

At 10% coating weight, the respective protection values for various coatings are summarized in the following table. The coating composition was (2M5VP/ST), talc, aluminum flake and stearic acid respectively in the proportions shown. Components in brackets were subjected to ball-mill treatment.

As previously described, the aluminum flake used in these experiments is made using stearic acid. Therefore, there is a stearic acid content from the aluminum flake. Thus, for example, although 5% by weight stearic acid was added, the composition actually contained 6% by weight stearic acid and this is reflected in the table.

| | Coating Composition | % Protection |
|---|---|---|
| Example 5 | 31.5/[38.1/24.4/6] | 88 |
| Example 6 | 31.5/[38.1/24.4]/6 | 87 |
| (Control) | 31.5/38.1/24.4/6 | 51 |
| Example 7 | 31.5/[41.1/26.4/1] | 77 |
| (Control) | 31.5/41.1/26.4/1 | 41 |

Coatings with ball-mill treatment show significantly higher protection values than controls.

## Example 8

Lysine.HCl/methionine (8/1) cores (−12/+16 sieve size) were coated with a formulation composed of (2M5VP/ST)/talc/aluminium/stearic acid (31.5/31.75/31.75/5). Talc, aluminum flake, and stearic acid were ball-milled in acetone and directly dispersed in the polymer solution in preparing the coating compositions with two levels of total solid contents. Results show that protection values for coated pellets with ball-mill-treated coatings are higher than for the controls. The protection values for pellets coated with the treated coating at 10% solids are even higher than the respective values for the control coating at 6% solids. These results clearly demonstrate that treatment of flake material with hydrophobic substances by ball milling improves the coating. Thus, ball-milling of flake material and hydrohobic substance allows the coating to be performed at a higher composition solid content level and provides as good protection as using the dilute coating compositions.

Unless otherwise specified, the compositions in which quantities are given (e.g., 31.5/38.1/24.4/6), indicate the percent by weight of the total weight of the coating composition of polymeric material, talc, aluminum flake and flake material, respectively.

Unless otherwise specified, all ratios, percentages, etc., are by weight.

## Claims

1. A method for making coated pellets comprising a core material and a coating, the coating composition comprising a film-forming polymeric material, a hydrophobic material and flake particles, said method comprising coating said core material with said coating composition, characterized in that prior to coating, said flake particles are brought into rubbing contact with said hydrophobic material.

2. Method according to Claim 1 wherein the flake material and hydrophobic material are ball-milled.

3. Method according to Claim 1 or 2 wherein the duration of said rubbing contact is from 2 to 28 hours.

4. Method according to Claim 3 wherein said rubbing contact is under light shearing stress of between 0.1 to 20 Kg/cm$^2$.

## Patentansprüche

1. Verfahren zur Herstellung von beschichteten Pellets mit einem Kernmaterial und einer Beschichtung, unter Verwendung einer Beschichtungsmasse mit einem filmbildenden polymeren Material, einem hydrophoben Material und schuppen-oder flockenförmigen Teilchen durch Beschichtung des Kernmaterials mit der Beschichtungsmasse, dadurch gekennzeichnet, daß man die flockenförmigen Teilchen vor der Beschichtung in reibenden Kontakt mit dem hydrophoben Material bringt.

2. Verfahren nach Anspruch 1, dadurch gekennzeichnet, daß man das schuppen- oder flockenförmige Material und das hydrophobe Material in einer Kugelmühle vermahlt.

3. Verfahren nach Anspruch 1 oder 2, dadurch gekennzeichnet, daß die Dauer des reibenden Kontaktes 2b is 28 Stunden beträgt.

4. Verfahren nach Anspruch 3, dadurch gekennzeichnet, daß der reibende Kontakt unter einer schwachen Scherbeannspruchung zwischen 0,1 bis 20 kg/cm² erfolgt.

**Revendications**

1. Procédé pour fabriquer des granulés enduits, comprenant un noyau et une couche dont la composition comprend une substance polymère filmogène, une substance hydrophobe et des particules sous forme de paillettes, ce procédé consistant à enduire le noyau de ces granulés d'une couche de cette composition, caractérisé en ce que, avant le couchage, on met les particules en paillettes en contact de frottement avec la substance hydrophobe.

2. Procédé conforme à la revendication 1, dans lequel les paillettes et la substance hydrophobe sont passées au broyeur à boulets.

3. Procédé conforme à la revendication 1 ou 2, dans lequel la durée de la mise en contact de frottement est de 2 h à 28 h.

4. Procédé conforme à la revendication 3, dans lequel la mise en contact de frottement a lieu sous une faible contrainte de cisaillement comprise entre 0,1 kg/cm² et 20 kg/cm².